# EUROPÄISCHE PATENTSCHRIFT

(11) **EP 3 859 954 B1**
(45) Veröffentlichungstag und Bekanntmachung des Hinweises auf die Patenterteilung: **30.09.2026**
(21) Anmeldenummer: 20154691.8
(22) Anmeldetag: 30.01.2020
(51) Int. Cl.: H02K 29/08, H02K 3/28, H02K 3/47, A61M 60/13, A61M 60/216, A61M 60/422, H02K 3/18

(54) **FUNKTIONSSICHERER BÜRSTENLOSER DC-ELEKTROMOTOR**
FUNCTIONALLY SAFE BRUSHLESS DC ELECTRIC MOTOR
MOTEUR ÉLECTRIQUE FIABLE À COURANT CONTINU SANS BALAIS

(43) Veröffentlichungstag der Anmeldung: 04.08.2021
(73) Patentinhaber: Maxon International AG, 6072 Sachseln (CH)
(72) Erfinder: Arend, Peter Ullrich, 5623 Boswil (CH); Boletis, Alexis, 6005 Luzern (CH)
(74) Vertreter: Grünecker Patent- und Rechtsanwälte PartG mbB

(56) Entgegenhaltungen:
- EP-A1- 1 940 007
- EP-A1- 3 016 247
- WO-A1-2018/166930
- WO-A1-84/03400
- CN-A- 102 594 077
- DE-T2- 69 629 255
- JP-U- H0 518 274
- US-A1- 2014 103 850
- US-A1- 2018 351 482
- US-A1- 2019 111 193

## Beschreibung

Die vorliegende Erfindung betrifft einen implantierbaren bürstenlosen DC-Elektromotor für eine Aktuatoreinheit eines Implantats, bevorzugt für ein Herzunterstützungssystem, mit einem Stator mit hohlzylindrischer eisenloser Wicklung und einem gegenüber dem Stator drehbaren Rotor, welcher eine Welle mit einer Anzahl p von permanentmagnetischen Polpaaren aufweist, wobei die Wicklung eine Anzahl n von voneinander getrennten 3-Phasensystemen aufweist.

Bürstenlose DC-Elektromotoren weisen eine von einem 3-Phasensystem mit Strom versorgte hohlzylindrische eisenlose Wicklung auf, die im Stator angeordnet ist, da kein Bürstensystem existiert, das den Strom auf eine rotierende Wicklung übertragen könnte. Entsprechend muss ein permanentmagnetisches Polpaar sich gegenüber dem Stator mit der Wicklung drehen. Dabei besteht das Material des permanentmagnetischen Polpaars üblicherweise aus einer Neodym-Eisen-Bor-Legierung, um eine ausreichend hohe Leistungsdichte zu erreichen. Die mit dem permanentmagnetischen Polpaar versehene Welle wird üblicherweise mittels vorgespannter Kugellager gelagert, um eine hohe Lebensdauer zu erreichen. Um eine höhere Leistungsdichte zu erreichen, kann der Rotor mit mehreren permanentmagnetischen Polpaaren versehen sein. Ein bürstenloser DC-Elektromotor benötigt zum Betrieb eine elektronische Kommutierung, die im Prinzip das Bürstensystem herkömmlicher DC-Elektromotoren imitiert. Entsprechend erhält man ein ähnliches Drehzahl-Drehmoment-Verhalten, wie bei einem herkömmlichen DC-Elektromotor mit einem großen Anlaufmoment und einer hohen Dynamik. Der Hauptvorteil eines bürstenlosen DC-Elektromotors sind die höhere Lebensdauer und die höhere Drehzahl, die nicht durch ein mechanisches Kommutierungssystem begrenzt sind. Insbesondere die hohlzylindrische eisenlose Wicklung ermöglicht einen bürstenlosen DC-Elektromotor mit reduzierten Eisenverlusten, mit niedriger Reibung und einer niedrigen Verlustwärme, wodurch sich ein äußerst effizienter Elektromotor ergibt, der aufgrund seiner geringen Trägheit eine hohe Beschleunigung und kurze Reaktionszeiten erlaubt, was insbesondere für die Verwendung bei Aktuatoreinheiten für Implantate, insbesondere für Herzunterstützungspumpen besonders vorteilhaft ist.

In den beiden letzten Dekaden ist der Einsatz bürstenloser DC-Elektromotoren stetig gestiegen, da die zum Betrieb benötigte Elektronik preiswerter und kleiner geworden ist, aber auch durch den gestiegenen Einsatz und die Ausweitung von Gebieten, in denen zuverlässige Elektromotoren mit einer Redundanz oder mit einer erhöhten Ausfallsicherheit unabdingbar sind.

Aus der EP 754 365 B1 ist ein redundanter Elektromotor bekannt, der einen Rotor mit zwei axial hintereinander angeordneten Magnetpolen sowie einen Stator aus zwei axial hintereinander angeordneten voneinander getrennten Wicklungen aufweist. Dabei sind die Wicklungen unabhängig voneinander ansteuerbar und jeweils einem permanentmagnetischen Polpaar zugeordnet. In der DE 31 40 034 A1 wird ein redundanter bürstenloser DC-Elektromotor beschrieben, bei dem die genuteten Statorwicklungen aus vier Teilsträngen bestehen, das Sensorsystem zur Kommutierung aus vier Sensorgruppen und der Rotor vier Polpaare aufweist. Somit weist dieser redundante Elektromotor vier separat ansteuerbare Motoreinheiten auf, durch die eine hohe Redundanz möglich ist, aber ein großes Bauvolumen und sehr viele einzelne Bauteile benötigt werden. Die JP H05 18274 U zeigt einen bürstenlosen Gleichstrommotor mit einem Rotor mit einer Vielzahl von Magnetpolpaaren und einem Stator mit einer Vielzahl in Nuten angeordneter dreiphasiger Spulen, wobei die axiale Länge des Stators kürzer als bei herkömmlichen miniaturisierten Motoren ist. Die EP 1 940 007 A1 beschreibt eine mehrphasige Statorwicklungsanordnung für einen Synchronmotor mit mehreren Spulengruppen und mehrere in Reihe geschaltete Statorspulen. Ein weiterer redundanter bürstenloser DC-Elektromotor ist aus der EP 2 922 180 A1 bekannt, bei dem die Wicklung des Stators mehrere voneinander unabhängig betreibbare Wicklungsstränge und mehrere unabhängige Sensorgruppen zur Erfassung der Drehposition des Rotors und zur elektronischen Kommutierung aufweist. Dabei ist die Spule als eine bifilare Wicklung ausgebildet, die mindestens zwei separat ansteuerbare Wicklungsstränge aus Einzeldrähten umfasst. Die bifilar gewickelte Spule bestehen zwar aus zwei parallel geführten Wicklungsdrähten, die von verschiedenen Spannungsquellen versorgt werden können, jedoch besteht im Falle eines Fehlers eine hohe Wahrscheinlichkeit, dass auch das direkt benachbarte redundante Wicklungssystem vor einer betrieblichen Temperaturerhöhung bzw. von einer fertigungstechnisch begründeten lokalen Schwächung betroffen ist. Außerdem wirkt sich im Falle eines Kurzschlusses der betroffene Wicklungsdraht magnetisch auf den bifilaren Nachbardraht derart aus, dass dieser ebenso wie der betroffene Wicklungsdraht von dem durch den entgegenwirkenden Kurzschluss-Strom reduzierten magnetischen Fluss erfasst wird und so nur reduziert an der elektromechanischen Energiewandlung teilnehmen kann. Entsprechend besitzt die bifilar gewickelte Spule eine geringere Redundanz bei einer hohen negativen Rückwirkung eines nicht abschaltbaren Kurzschluss-Stroms auf die verbleibende Wicklung.

Neben den langjährigen Einsatzgebieten redundanter Elektromotoren im Bereich der Luft- und Raumfahrt sind in den letzten Jahren viele implantierbare medizinische Geräte entwickelt worden, die ausfallsicher elektrisch betrieben werden müssen, beispielsweise Herzunterstützungspumpen. Die DE 696 29 255 T2 betrifft mechanische Blutpumpen mit einem bürstenlosen Stangenmotor zum Fördern von Blut zwischen permanentmagnetischem Rotor und Stator mit eisenlosen Statorwicklungen. Die US 2019/111193 A1 offenbart eine weitere Blutpumpe mit einem eisenlosen Stator, einer Rotorbaugruppe sowie einem radialen Motorluftspalt zwischen Stator und Rotor, durch den das Blut strömt. Gerade bei derartigen ventrikulären Hilfsgeräten ist ein sicherer unterbrechungsfreier Betrieb notwendig, da eine Unterbrechung oder ein signifikanter Leistungsabfall lebensbedrohliche Folgen haben kann. Neben der Redundanz der Antriebseinheit eines Implantats sind hier jedoch auch eine geringe Baugröße und ein geringes Gewicht der Antriebseinheit notwendige Aspekte, um einen Einsatz in einem Implantat zu ermöglichen. Der vorliegenden Erfindung liegt daher die Aufgabe zugrunde, einen funktionssicheren bürstenlosen DC-Elektromotor bereitzustellen, der bei einer leichten und kompakten Konstruktion eine ausreichende Redundanz aufweist, um bei einem lokalen Fehler einen Betrieb zumindest beschränkt aufrechtzuerhalten.

Diese Aufgabe wird erfindungsgemäß durch einen implantierbaren bürstenlosen DC-Elektromotor gemäß dem beigefügten Satz von Ansprüchen gelöst. Die räumlich versetzte Anordnung der Vielzahl von elektrisch voneinander getrennten 3-Phasensysteme in den voneinander getrennten, d.h. elektrisch isolierten, Wicklungssträngen, führt zu einer Segmentierung der um den Rotor angeordneten hohlzylindrischen eisenlosen Wicklung und damit ermöglicht eine fehlertolerante Wicklungsverschaltung eines bürstenlosen DC-Elektromotors. Bei einer Anzahl p > 1 von permanentmagnetischen Polpaaren lassen sich einem oder mehreren Polpaaren bestehend aus einem Nord- und Südpol mindestens ein getrenntes 3-Phasensystem zuordnen, wodurch eine Entkopplung der magnetischen Kreise erreicht wird, sodass bei einem Kurzschluss in einem der getrennten 3-Phasensysteme keine bzw. eine reduzierte magnetische Auswirkung auf die weiteren 3-Phasensysteme auftritt.

Der Einsatz von mindestens zwei räumlich voneinander getrennten 3-Phasensystemen in der hohlzylindrischen eisenlosen Wicklung eines erfindungsgemäßen bürstenlosen DC-Elektromotors ermöglicht nicht nur ein kleines und kompaktes Motordesign mit geringer Baulänge und geringem Durchmesser bei einer gleichzeitig hohen Nennleistung, sondern auch eine hohe Redundanz gegenüber Fehlern im Wicklungssystem, beispielsweise aufgrund einer betrieblichen Temperaturerhöhung oder einer fertigungstechnisch begründeten lokalen Schwächung der Wicklung, weshalb dieser bürstenlose DC-Elektromotor hervorragend für eine Antriebseinheit eines Implantats geeignet ist, beispielsweise in einem implantierbaren Herzunterstützungssystem zur längerfristigen Unterstützung des Blutkreislaufes des menschlichen Herzens. Bei solchen implantierbaren Herzunterstützungssystemen können mögliche Fehler in einer Spule der Wicklung, aber auch Fehler in der Zuleitung oder der Verbindung zur Zuleitung, zu einem Ausfall des Herzunterstützungssystems und damit zum Tod des Patienten führen.

Erfindungsgemäß ist für jede Phase der voneinander elektrisch getrennten 3-Phasensysteme mindestens eine Anzahl von k = 2 Einzelspulen in Reihe geschaltet sind, wobei ein Produkt k·n aus den k Einzelspulen und den n getrennten Phasensystemen einem Zweifachen der permanentmagnetischen Polpaare entspricht und wobei die in Reihe geschalteten Einzelspulen der jeweiligen Phase der voneinander getrennten 3-Phasensysteme um den räumlichen Winkel 360°/n/k zueinander versetzt angeordnet sind. Die Gesamtanzahl der Einzelspulen des Stators entspricht somit dem Zweifachen der permanentmagnetischen Polpaare bzw. der Gesamtanzahl von Rotorpolen, so dass der räumliche Winkel zwischen zwei Einzelspulen dem räumlichen Winkel zwischen zwei Rotorpolen entspricht. Die räumlich voneinander getrennte Anordnung der Einzelspulen erhöht die Fehlertoleranz des Elektromotors beim Ausfall eines Wicklungsstrangs und verbessert damit die Nutzbarkeit des Elektromotors als Antriebseinheit eines Implantats, für das eine hohe Ausfallsicherheit notwendig ist. Bevorzugt wird dabei der DC-Elektromotor als vierpoliger permanenterregter Synchronmotor mit zwei elektrisch voneinander getrennten 3-Phasensystemen ausgeführt, bei dem jede Phase der 3-Phasensysteme zwei in Reihe geschaltete und in einem räumlichen Winkel von 90° angeordnete Einzelspulen aufweist. Mit einem solchen vierpoligen Synchronmotor kann bereits eine hohe Redundanz bei geringer Baugröße und Herstellungsaufwand erzielt werden. Um eine möglichst große Windungsdichte der hohlzylindrischen eisenlosen Wicklung zu erzielen, sind die Einzelspulen der 3-Phasensysteme mit einer rhombenförmigen Wicklung versehen, die auch als Rautenwicklung bezeichnet wird.

Für eine erhöhte Fehlertoleranz können jeweils zwei in Reihe geschaltete Einzelspulen einer Phase der mindestens zwei voneinander getrennten 3-Phasensysteme in der hohlzylindrischen eisenlosen Wicklung in entgegengesetzter Wicklungsrichtung elektrisch verbunden sein. Die Verschaltung von zwei Einzelspulen in entgegengesetzter Wicklungsrichtung führt zu einer magnetischen Spulenkopplung mit einem Nord- und einem Südpol, wodurch in einem Notfallbetrieb die Wicklungsströme reduziert werden.

Eine zweckmäßige Ausbildung sieht vor, dass die axialen Positionen der mindestens zwei voneinander getrennten 3-Phasensysteme gegenüber der Welle sich zumindest bereichsweise überlappen. Die überlappende Anordnung der axialen Positionen der mindestens zwei elektrisch voneinander getrennten 3-Phasensysteme in Richtung der axialen Erstreckung der Welle ermöglicht eine kompakte Bauweise des bürstenlosen DC-Elektromotors mit einer geringen Gesamtlänge. Dabei ist es ausreichend, wenn sich die Magnetfelder der 3-Phasensysteme über eine gewisse axiale Länge auf einen gemeinsamen Abschnitt der Welle wirken. Bevorzugt wirken die sich senkrecht zur axialen Richtung der Welle erstreckenden Magnetfelder der 3-Phasensysteme im Wesentlichen nur auf einen gemeinsamen Abschnitt der Welle, um trotz einer hohen Redundanz eine sehr kurze Motorlänge zu ermöglichen.

Eine sinnvolle Ausgestaltung sieht vor, dass die Einzelspulen der voneinander getrennten 3-Phasensysteme in einer Sternschaltung miteinander verbunden sind, wobei die Sternpunkte der mindestens zwei voneinander getrennten 3-Phasensysteme bevorzugt miteinander verbunden sind. Der Einsatz einer Sternschaltung zur elektrischen Kopplung der Einzelspulen der jeweiligen 3-Phasensysteme ermöglicht neben einer höheren Drehmomentkonstante auch die Vermeidung von unerwünschten Kreisströmen in der Wicklung. Dabei können die Sternpunkte in einfacher Weise einzeln oder als gemeinsamer Sternpunkt aus der hohlzylindrischen eisenlosen Wicklung herausgeführt und an die Leistungselektronik angeschlossen werden.

Alternativ sind die Einzelspulen der einzelnen voneinander getrennten 3-Phasensysteme in Reihe miteinander verbunden, sodass eine als Dreieckschaltung bezeichnete elektrische Kopplung der einzelnen 3-Phasensysteme entsteht. Die Dreieckschaltungen können dann jeweils an die Leistungselektronik angeschlossen werden. Alternativ sind die Einzelspulen der voneinander getrennten 3-Phasensystemen eines erfindungsgemäßen bürstenlosen DC-Elektromotors in einer einzigen Polygonschaltung miteinander verbunden. Die Dreiecksschaltung der Einzelspulen bzw. die Polygonschaltung der Einzelspulen mehrerer 3-Phasensysteme ermöglicht eine höhere Drehzahlkonstante des Elektromotors und damit eine Reduzierung der notwendigen Versorgungsspannung.

Günstigerweise kann für jede der voneinander getrennten 3-Phasensysteme ein separater elektronischer Kommutator vorgesehen sein, bevorzugt ein elektronischer Blockkommutator. Dadurch können trotz der Verwendung von zwei elektrisch und räumlich voneinander getrennten 3-Phasensystemen herkömmliche und auch für Standardmotoren verwendete Kommutatoren eingesetzt werden. Entsprechend lassen sich so die Entwicklungs- und Produktionskosten redundanter bürstenloser DC-Elektromotoren senken. Weiterhin entsteht beim Einsatz eines separaten elektronischen Kommutators für jedes der voneinander getrennten 3-Phasensysteme bei dem Ausfall eines Kommutators nur eine minimale Beeinträchtigung der 3-Phasensysteme untereinander, sofern die Kommutatoren mit eigenen Spannungskreisen ausgestattet sind. Folglich kann so die Fehlertoleranz des erfindungsgemäßen DC-Elektromotors verbessert und eine erhöhte Redundanz erzielt werden.

Des Weiteren kann der Stator einen magnetischen Rückschluss aufweisen, bevorzugt ein um die hohlzylindrische eisenlose Wicklung herum angeordnetes laminiertes Eisenpaket. Der magnetische Rückschluss reduziert die Wirbelstromverluste und verbessert entsprechend die Leistungsdichte des DC-Elektromotors. Für einen möglichst hochwertigen Rückschluss kann ein laminiertes Eisenpaket oder ein Paket aus Eisen-Nickel-Blechen um die hohlzylindrische eisenlose Wicklung herum angeordnet sein.

Eine besondere Ausführungsform sieht vor, dass der Luftspalt zwischen dem Rotor und dem Stator eine Fluidströmung durch den Luftspalt ermöglicht, insbesondere eine Durchströmung von menschlichem Blut ermöglicht, wobei der umlaufende Luftspalt bevorzugt größer als 15%, insbesondere größer als 25%, des Radius des Rotors ist. Ein derart großer Luftspalt zwischen dem Rotor und dem Stator ermöglicht einen einfachen Einsatz als Antriebseinheit eines implantierbaren Herzunterstützungssystems und dessen Integration in dem Blutkreislauf von Patienten.

Im Folgenden werden nichtbeschränkende Ausführungsbeispiele der vorliegenden Erfindung anhand von beispielhaften Zeichnungen näher erläutert. Es zeigen:
- Fig. 1: eine schematische Explosionsdarstellung eines erfindungsgemäßen bürstenlosen DC-Elektromotors,
- Fig. 2: eine schematische Darstellung der Spulenverschaltung des DC-Elektromotors aus Fig. 1 mit zwei permanentmagnetischen Polpaaren und zwei 3-Phasensystemen,
- Fig. 3: eine schematische Darstellung der unabhängigen magnetischen Flüsse für die Schaltung aus Fig. 2 mit zwei Einzelspulen je Phase,
- Fig. 4: eine schematische Darstellung der unabhängigen magnetischen Flüsse entsprechend Fig. 3 mit einem internen Windungsschluss, und
- Fig. 5: eine schematische Darstellung einer Wicklungsausführung für die Schaltung aus Fig. 2 mit zwei Einzelspulen je Phase.

Für die folgenden Ausführungsbeispiele gilt, dass gleiche Teile durch gleiche Bezugszeichen bezeichnet sind. Sofern in einer Figur Bezugszeichen enthalten sind, auf die in der zugehörigen Figurenbeschreibung nicht näher eingegangen wird, so wird auf vorangehende oder nachfolgende Figurenbeschreibungen verwiesen.

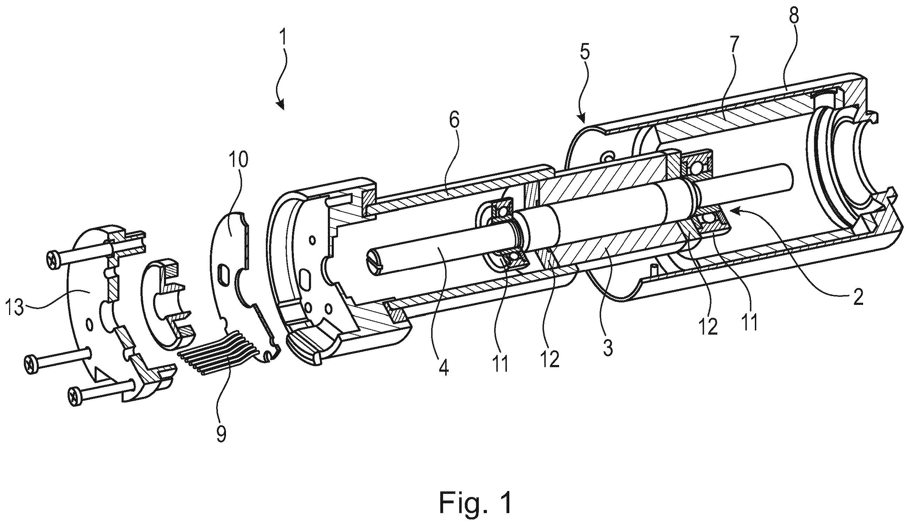

Anhand der Fig. 1 wird zunächst der generelle Aufbau eines erfindungsgemäßen bürstenlosen DC-Elektromotors 1 erläutert. Die wesentlichen Bestandteile dieses bürstenlosen DC-Elektromotors 1 sind der drehbare Rotor 2 mit einem Permanentmagneten 3, der direkt mit der Welle 4 verbunden ist, sowie der Stator 5, in dem der Rotor 2 drehbar gelagert ist, mit einer hohlzylindrischen eisenlosen Wicklung 6 und dem um die Wicklung 6 herum angeordneten mit dem Gehäuse 8 verbundenen Rückschluss 7. Dabei besteht der Rückschluss 7 aus einem laminierten Eisenpaket, um die Eisenverluste zu verkleinern, die aufgrund des rotierenden Permanentmagneten 3 des Rotors 2 auftreten. Eine Printplatte 10 sorgt über Anschlussdrähte 9 für die elektrische Verbindung der Wicklung 6 mit einer zugehörigen Leistungselektronik. Dabei können auf der Printplatte 10 auch Sensoren, beispielsweise Hallsensoren, angeordnet sein, welche die Lage der mit der Welle 4 umlaufenden Permanentmagneten 3 abtasten.

Die Welle 4 mit dem darauf montierten Permanentmagneten 3 ist auf zwei vorgespannten Kugellagern 11 drehbar gelagert. Zwei zwischen den Kugellagern 11 und dem Permanentmagneten 3 angeordnete Auswuchtringe 12 ermöglichen ein dynamisches Auswuchten des Rotors, indem gezielt Material von den beiden Auswuchtringen 12 entfernt werden kann. Das Auswuchten des Rotors mittels der Auswuchtringe 12 reduziert die Vibration und Geräusche und verlängert damit die Lebensdauer der Kugellager 11, bzw. des gesamten Elektromotors 1, insbesondere bei den mit einem bürstenlosen DC-Elektromotor 1 erreichbaren hohen Drehzahlen. Mit dem stirnseitigen Lagerflansch 13 kann der im Gehäuse 8 montierte Rotor 2 gesichert werden.

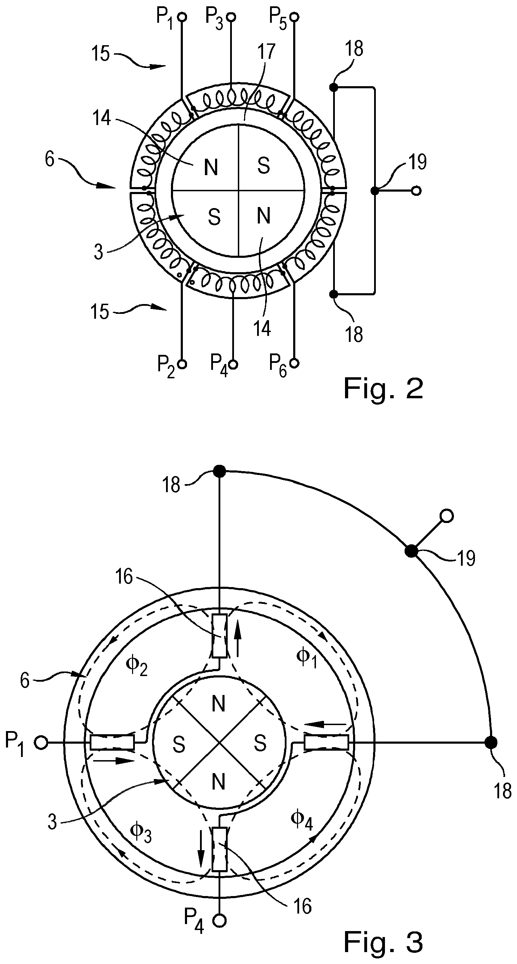

Die Verschaltung der hohlzylindrischen eisenlosen Wicklung 6 des DC-Elektromotors 1 aus Fig. 1 mit einem Permanentmagneten 3 aus zwei permanentmagnetischen Polpaaren 14 sowie zwei elektrisch voneinander isolierte 3-Phasensystemen 15 der Wicklung 6 mit den Einzelphasen P₁, P₃ und P₅ bzw. P₂, P₄ und P₆ ist in Fig. 2 dargestellt. Neben dem Permanentmagneten 3 mit vier magnetischen Polen weisen die Einzelphasen P₁, P₃ und P₅ bzw. P₂, P₄ und P₆ jeweils zwei Einzelspulen 16 auf, die in Reihe geschaltet und um einen räumlichen Winkel von 90° zueinander versetzt in der Wicklung 6 angeordnet sind. Dabei ist zwischen dem Stator 5 mit der Wicklung 6 und dem Rotor 2 mit dem Permanentmagneten 3 ein größerer Luftspalt 17 vorgesehen, der eine Fluidströmung, insbesondere eine Durchströmung mit menschlichem Blut, erlaubt. Weiter sind die Einzelphasen P₁, P₃ und P₅ bzw. P₂, P₄ und P₆ der jeweiligen 3-Phasensysteme 15 elektrisch miteinander in einer Sternschaltung verbunden, wobei die einzelnen Sternpunkte 18 der zwei 3-Phasensystemen 15 miteinander verschmolzen und als gemeinsamer Sternpunkt 19 aus der Wicklung 6 herausgeführt sind. Alternativ zu der in Fig.2 gezeigten Sternschaltung lassen sich die zwei 3-Phasensystemen 15 entsprechend auch als eine 6-phasige Polygonschaltung verbinden, ohne einzelne Sternpunkte 18 oder einen gemeinsamen Sternpunkt 19.

Fig. 3 zeigt eine schematische Darstellung der unabhängigen magnetischen Flüsse für einen erfindungsgemäßen DC-Elektromotor 1 in der in Fig. 2 gezeigten Sternschaltung. Dabei ist auch hier wieder die bevorzugte Ausführungsform des DC-Elektromotors 1 mit einem 4-poligen Permanentmagneten 3 und zwei 3-Phasensystemen 15 mit jeweils 2 in Reihe geschalteten Einzelspulen 16 pro Phase, welche in einem räumlichen Winkel von 90° zueinander in der Wicklung 6 angeordnet sind, zugrunde gelegt. Fig. 3 zeigt das Schema der unabhängigen magnetischen Flüsse Φ₁ bis Φ₄ der vier Einzelspulen 16 von zwei zueinander gehörenden, d.h. um 180° versetzten einzelnen Phasen der zwei voneinander getrennten 3-Phasensystemen 15. Dabei sind die zwei Einzelspulen 16 der jeweiligen einzelnen Phasen in entgegengesetzter Wicklungsrichtung elektrisch verschaltet, was zu einer magnetischen Spulenkopplung mit einem Nord- und einem Südpol führt.

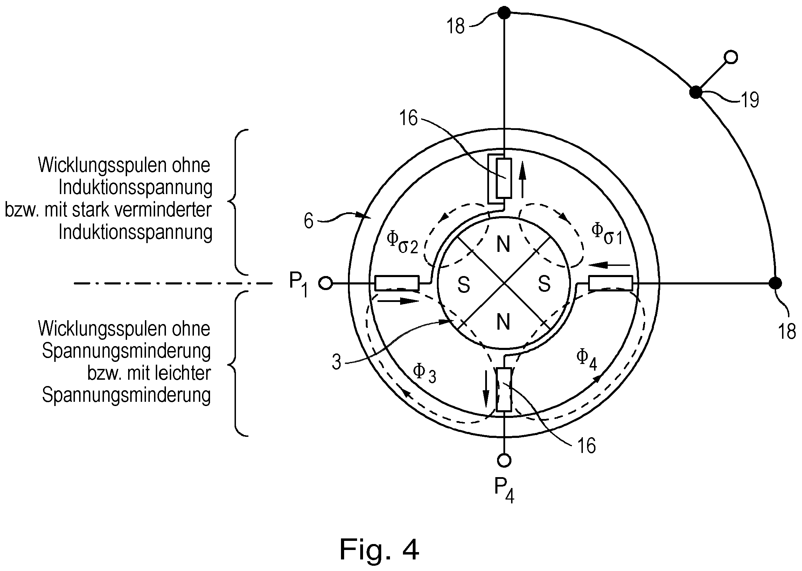

Die schematische Darstellung in Fig. 4 zeigt das Schema der unabhängigen magnetischen Flüsse Φ₁ bis Φ₄ der vier Einzelspulen 16 für einen fehlerbehafteten Betriebszustand in dem eine Einzelspule 16 der Wicklung 6 durch einen internen Wicklungsfehler kurzgeschlossen ist und ideell betrachtet keinen magnetischen Fluss zulässt. Im Gegensatz zu dem ungestörten Betriebszustand des DC-Elektromotors 1 in Fig. 3 ist eine Einzelspule 16 der Einzelphase P₁ in unvorhergesehener Weise durch einen internen Windungsschluss kurzgeschlossen, wodurch sich ein Kurzschluss-Strom ausbildet, der dem Rotorfeld entgegenwirkt. Die magnetischen Flüsse Φ₁ und Φ₂ werden aus der kurzgeschlossenen Einzelspule 16 der Einzelphase P₁ heraus gedrängt und müssen sich daher tangential, d.h. in Umfangsrichtung, im Luftspalt 17 zwischen der Oberfläche des Rotors 2 und dem inneren Durchmesser der Wicklung 6 schließen. Die magnetischen Flüsse Φ₁ und Φ₂ bilden somit lediglich magnetische Streufluss-Komponenten aus, die von dem Wicklungssystem nicht mehr erfassen werden und damit auch nicht mehr zum elektromechanischen Leistungsumsatz beitragen können.

Der magnetische Spulenfluss bestehend aus den magnetischen Flüssen Φ₁ und Φ₂ der Einzelphase P₁ ist durch den Windungsschluss um 3/4 auf 1/4 reduziert. Der magnetische Spulenfluss in der gegenüberliegenden Einzelphase P₂ der Wicklung 6 zeigt jedoch lediglich eine Minderung um 1/4 auf 3/4 des Flusses, der in dem fehlerfreien Zustand aus Fig. 3 herrschte. Nach dem Wegschalten der kurzschlussbehafteten Einzelphase P₁ kann die Einzelphase P₂ mit um 25% reduziertem Fluss noch weiter betrieben werden. Diese relativ kleine Beeinträchtigung ist der Tatsache geschuldet, dass den Einzelphasen direkt benachbarte Polpaare zugeordnet sind. So können nach dem Wegschalten einer fehlerhaften Einzelphase immer noch 10/12, also etwa 83%, des Wicklungssystems einen Leistungsbeitrag leisten. Entsprechend können im Notfallbetrieb die Wicklungsströme und entsprechend dem Strom-Quadrat auch die Kupferverluste reduziert werden. Wegen des höheren Wirkungsgrades im Notfallbetrieb ist der erfindungsgemäße DC-Elektromotor 1 als deutlich fehlertoleranter einzustufen als herkömmliche Elektromotoren mit einer einfachen 3-phasigen Wicklung 6. Auch ist mit dem erfindungsgemäßen DC-Elektromotor 1 im Notfallbetrieb ein Anlauf aus dem Stillstand heraus weiterhin möglich.

Die Mehrphasigkeit des erfindungsgemäßen DC-Elektromotors 1 mit kleineren Spannungsdifferenzen zwischen benachbarten Einzelspulen 16 reduziert bei einem Notfallbetrieb mit inneren Windungsschlüssen den Strom zwischen benachbarten Einzelphasen P₁ bis P₆. Der Notfallbetrieb bei einem bestehenden internen Windungsschluss verursacht somit geringere zusätzliche Verluste und entsprechend ermöglicht der erfindungsgemäße DC-Elektromotor 1 einen besseren Wirkungsgrad im Notfallbetrieb. Gerade im Gegensatz zu bifilar gewickelten Wicklungen 6 kann sich beim erfindungsgemäßen DC-Elektromotor 1 bei einem internen Windungsschluss immer noch ein magnetischer Fluss auf über der Hälfte des Umfangs der Wicklung 6 ausbilden, der in den verbleibenden, nicht betroffenen Einzelspulen 16eine leicht reduzierte Spannungsinduktion ermöglicht.

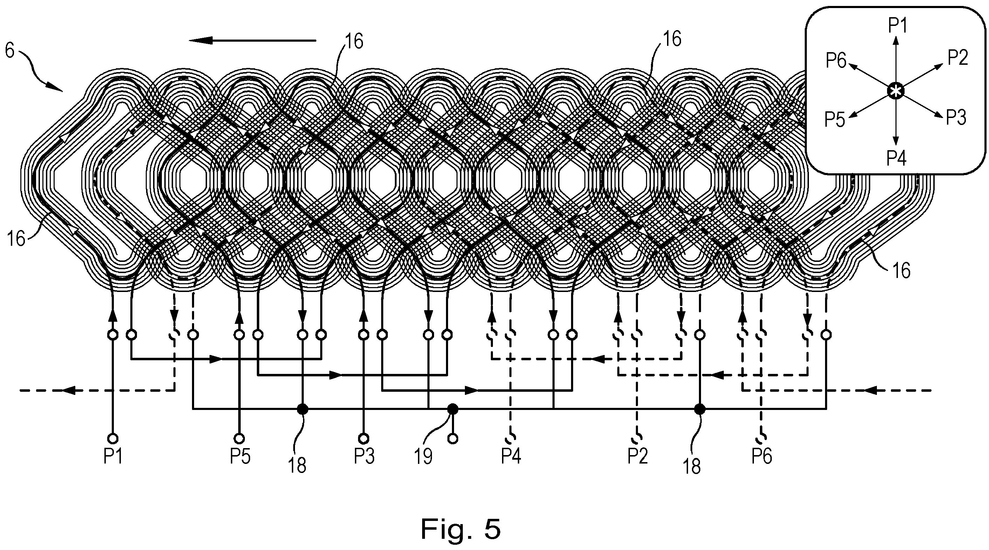

Die Wicklungsausführung für den in Fig. 2 dargestellten erfindungsgemäßen DC-Elektromotor 1 wird in der schematischen Darstellung der Wicklung 6 mit zwei Einzelspulen 16 je Einzelphase P₁ bis P₆ in Fig. 5 gezeigt. Dabei sind die Einzelphasen P₁ bis P₆ der zwei voneinander getrennten 3-Phasensysteme 15 in einer Sternschaltung miteinander verbunden, wobei die Sternpunkte 18 der mindestens zwei voneinander getrennten 3-Phasensysteme 15 miteinander verbunden sind und als gemeinsamer Sternpunkt 19 aus der Wicklung 6 herausgeführt und an eine Leistungselektronik angeschlossen werden können. Die Einzelspulen 16 der zwei 3-Phasensysteme 15 sind in einer rhombenförmigen Wicklungsform ausgebildet, um eine möglichst große Windungsdichte der hohlzylindrischen eisenlosen Wicklung 6 zu erzielen.

Diese Wicklungsform, wird auch als Rautenwicklung bezeichnet. Die zwei Einzelspulen 16 jeweiligen Einzelphasen P₁ bis P₆ sind in der hohlzylindrischen eisenlosen Wicklung in einer entgegengesetzten Wicklungsrichtung elektrisch verbunden, was zu einer magnetischen Spulenkopplung mit einem Nord- und einem Südpol und damit zu der erhöhten Fehlertoleranz des erfindungsgemäßen DC-Elektromotors 1 führt.

### Bezugszeichenliste

- 1: DC-Elektromotor
- 2: Rotor
- 3: Permanentmagnet
- 4: Welle
- 5: Stator
- 6: Wicklung
- 7: Rückschluss
- 8: Gehäuse
- 9: Anschlussdrähte
- 10: Printplatte
- 11: Kugellager
- 12: Auswuchtringe
- 13: Lagerflansch
- 14: Polpaar
- 15: 3-Phasensystem
- 16: Einzelspulen
- 17: Luftspalt
- 18: Sternpunkte
- 19: gemeinsamer Sternpunkt

- P₁-P₆: Einzelphasen
- Φ₁-Φ₄: magnetische Flüsse

## Patentansprüche

1. Implantierbarer bürstenloser DC-Elektromotor (1) für eine Aktuatoreinheit eines Implantats, bevorzugt für ein Herzunterstützungssystem, mit einem Stator (5) mit hohlzylindrischer eisenloser Wicklung (6) und einem gegenüber dem Stator (5) drehbaren Rotor (2), welcher eine Welle (4) mit einer Anzahl **p** von permanentmagnetischen Polpaaren (14) aufweist, wobei die Wicklung (6) eine Anzahl **n** von voneinander getrennten 3-Phasensystemen aufweist und die Anzahl n der voneinander getrennten 3-Phasensysteme (15) in der hohlzylindrischen eisenlosen Wicklung (6) räumlich um einen Winkel von 360°/**n** versetzt zueinander angeordnet ist, und wobei für jede Phase der voneinander getrennten 3-Phasensysteme (15) mindestens eine Anzahl von **k**=2 Einzelspulen (16) in Reihe geschaltet sind,
**dadurch gekennzeichnet, dass** die Anzahl **n** der voneinander getrennten 3-Phasensystemen (15) der doppelten Anzahl **p** von permanentmagnetischen Polpaaren (14) entspricht,
wobei ein Produkt **k·n** einem Zweifachen der Anzahl **p** entspricht und wobei die in Reihe geschalteten Einzelspulen (16) der jeweiligen Phase der voneinander getrennten 3-Phasensysteme (15) um den räumlichen Winkel 360°/**n/k** zueinander versetzt angeordnet sind.

2. Implantierbarer bürstenloser DC-Elektromotor (1) nach Anspruch 1,
**dadurch gekennzeichnet, dass** jeweils zwei in Reihe geschaltete Einzelspulen (16) einer Phase in der hohlzylindrischen eisenlosen Wicklung (6) in entgegengesetzter Wicklungsrichtung elektrisch verbunden sind.

3. Implantierbarer bürstenloser DC-Elektromotor (1) nach einem der Ansprüche 1 bis 2,
**dadurch gekennzeichnet, dass** die axialen Positionen der mindestens zwei voneinander getrennten 3-Phasensysteme (15) gegenüber der Welle (4) sich zumindest bereichsweise überlappen.

4. Implantierbarer bürstenloser DC-Elektromotor (1) nach einem der Ansprüche 1 bis 3,
**dadurch gekennzeichnet, dass** die Einzelspulen (16) der voneinander getrennten 3-Phasensysteme (15) in einer Sternschaltung miteinander verbunden sind, wobei die Sternpunkte (18) der mindestens 2 voneinander getrennten 3-Phasensysteme bevorzugt miteinander verbunden sind.

5. Implantierbarer bBürstenloser DC-Elektromotor (1) nach einem der Ansprüche 1 bis 4,
**dadurch gekennzeichnet, dass** die Einzelspulen (16) der einzelnen voneinander getrennten 3-Phasensysteme (15) in Reihe miteinander verbunden sind, sodass eine als Dreieckschaltung bezeichnete elektrische Kopplung der einzelnen 3-Phasensystems (15) entsteht.

6. Implantierbarer bürstenloser DC-Elektromotor (1) nach einem der Ansprüche 1 bis 5,
**dadurch gekennzeichnet, dass** die Einzelspulen der voneinander getrennten 3-Phasensystemen (15) eines erfindungsgemäßen bürstenlosen DC-Elektromotors (1) in einer einzigen Polygonschaltung miteinander verbunden sind.

7. Implantierbarer bürstenloser DC-Elektromotor (1) nach einem der Ansprüche 1 bis 6,
**dadurch gekennzeichnet, dass** für jede der voneinander getrennten 3-Phasensysteme (15) ein separater elektronischer Kommutator vorgesehen ist, bevorzugt ein elektronischer Blockkommutator.

8. Implantierbarer bürstenloser DC-Elektromotor (1) nach einem der Ansprüche 1 bis 7,
**dadurch gekennzeichnet, dass** der Stator (5) einen magnetischen Rückschluss (7) aufweist, bevorzugt ein um die hohlzylindrische eisenlose Wicklung (6) herum angeordnetes laminiertes Eisenpaket.

9. Implantierbarer bürstenloser DC-Elektromotor nach einem der Ansprüche 1 bis 8,
**dadurch gekennzeichnet, dass** der Luftspalt (17) zwischen dem Rotor (2) und dem Stator (5) eine Fluidströmung durch den Luftspalt (17) ermöglicht, insbesondere eine Durchströmung von menschlichem Blut ermöglicht, wobei der umlaufende Luftspalt (17) bevorzugt größer als 15%, insbesondere größer als 25%, des Radius des Rotors (2) ist.

## Claims

1. An implantable brushless DC motor (1) for an actuator unit of an implant, preferably for a cardiac assist system, comprising a stator (5) with a hollow cylindrical ironless winding (6) and a rotor (2) rotatable relative to the stator (5), which comprises a shaft (4) with a number **p** of permanent magnetic pole pairs (14), wherein the winding (6) comprises a number **n** of separate three-phase systems, and the number n of the separate three-phase systems (15) in the hollow cylindrical ironless winding (6) are spatially offset from one another by an angle of 360°/n, and wherein for each phase of the separate three-phase systems (15), at least a number of **k**=2 individual coils (16) are connected in series,,
**characterized in that** the number n of the separate three-phase systems (15) corresponds to twice the number **p** of permanent magnetic pole pairs (14),
where the product **k·n** corresponds to twice the number p, and wherein the individual coils (16) connected in series for the respective phase of the separate three-phase systems (15) are spatially offset from one another by a spatial angle of 360°/ **n/k.**

2. The implantable brushless DC motor (1) according to claim 1,
**characterized in that** two individual coils (16) of a phase, connected in series, are electrically connected in opposite winding directions within the hollow cylindrical ironless winding (6).

3. The implantable brushless DC motor (1) according to any one of claims 1 to 2,
**characterized in that** the axial positions of the at least two separate 3-phase systems (15) rela-tive to the shaft (4) partially overlap.

4. The implantable brushless DC motor (1) according to any one of claims 1 to 3,
**characterized in that** the individual coils (16) of the separate three-phase systems (15) are connected to one another in a star connection, wherein the star points (18) of the at least two separate three-phase systems are preferably connected to one another.

5. The implantable brushless DC motor (1) according to any one of claims 1 to 4,
**characterized in that** the individual coils (16) of the separate three-phase systems (15) are connected in series with one another, thereby creating an electrical coupling of the individual three-phase systems (15) known as a delta connection.

6. The implantable brushless DC motor (1) according to any one of claims 1 to 5,
**characterized in that** the individual coils of the separate three-phase systems (15) of an im-plantable brushless DC motor (1) according to the invention are connected to one another in a single polygon connection.

7. The implantable brushless DC motor (1) according to any one of claims 1 to 6,
**characterized in that** a separate electronic commutator, preferably an electronic block commutator, is provided for each of the separate three-phase systems (15).

8. The implantable brushless DC motor (1) according to any one of claims 1 to 7,
**characterized in that** the stator (5) comprises a magnetic return path (7), preferably a laminated iron core arranged around the hollow cylindrical ironless winding (6).

9. The implantable brushless DC motor according to any one of claims 1 to 8,
**characterized in that** the air gap (17) between the rotor (2) and the stator (5) allows for fluid flow through the air gap (17), in particular allowing for the flow of human blood, wherein the circumferential air gap (17) is preferably greater than 15%, in particular greater than 25%, of the radius of the rotor (2).

## Revendications

1. Moteur électrique à courant continu sans balais implantable (1) pour une unité d'actionnement d'un implant, de manière préférée pour un système d'assistance cardiaque, comprenant un stator (5) avec un enroulement (6) cylindrique creux sans fer et un rotor (2) rotatif par rapport au stator (5), qui présente un arbre (4) avec un nombre **p** de paires de pôles (14) à magnétisme permanent, dans lequel l'enroulement (6) présente un nombre **n** de systèmes triphasés séparés les uns des autres et le nombre **n** de systèmes triphasés (15) séparés les uns des autres sont agencés de manière spatialement décalée au sein de l'enroulement (6) cylindrique creux sans fer selon un angle de 360°/**n** les uns par rapport aux autres, et dans lequel au moins un nombre de **k**=2 bobines individuelles (16) sont connectées en série pour chaque phase des systèmes triphasés (15) séparés les uns des autres,
**caractérisé en ce que** le nombre **n** de systèmes triphasés (15) séparés les uns des autres correspond au double du nombre **p** de paires de pôles à magnétisme permanent (14),
dans lequel un produit **k·n** correspond à deux fois le nombre **p** et dans lequel les bobines individuelles (16) connectées en série de la phase respective des systèmes triphasés (15) séparés les uns des autres sont agencées de manière décalée les unes par rapport aux autres selon un angle spatial de 360°/**n/k**.

2. Moteur électrique à courant continu sans balais implantable (1) selon la revendication 1,
**caractérisé en ce que** respectivement deux bobines individuelles (16) connectées en série d'une phase sont connectées électriquement dans le sens opposé d'enroulement au sein de l'enroulement (6) cylindrique creux sans fer.

3. Moteur électrique à courant continu sans balais implantable (1) selon l'une quelconque des revendications 1 à 2,
**caractérisé en ce que** les positions axiales des au moins deux systèmes triphasés (15) séparés les uns des autres se chevauchent au moins par endroits par rapport à l'arbre (4).

4. Moteur électrique à courant continu sans balais implantable (1) selon l'une quelconque des revendications 1 à 3,
**caractérisé en ce que** les bobines individuelles (16) des systèmes triphasés (15) séparés les uns des autres sont connectées les unes aux autres en un circuit en étoile, dans lequel les points d'étoile (18) des au moins 2 systèmes triphasés séparés les uns des autres sont de manière préférée connectés les uns aux autres.

5. Moteur électrique à courant continu sans balais implantable (1) selon l'une quelconque des revendications 1 à 4,
**caractérisé en ce que** les bobines individuelles (16) des différents systèmes triphasés (15) séparés les uns des autres sont connectées les unes aux autres en série, de sorte qu'un couplage électrique, appelé circuit triangulaire, des différents systèmes triphasés (15) est créé.

6. Moteur électrique à courant continu sans balais implantable (1) selon l'une quelconque des revendications 1 à 5,
**caractérisé en ce que** les bobines individuelles des systèmes triphasés (15) séparés les uns des autres d'un moteur électrique à courant continu sans balais (1) selon la présente invention sont connectées les unes aux autres au sein d'un unique circuit polygonal.

7. Moteur électrique à courant continu sans balais implantable (1) selon l'une quelconque des revendications 1 à 6,
**caractérisé en ce qu'**un commutateur électronique séparé, de manière préférée un commutateur électronique à bloc, est fourni pour chacun des systèmes triphasés (15) séparés les uns des autres.

8. Moteur électrique à courant continu sans balais implantable (1) selon l'une quelconque des revendications 1 à 7,
**caractérisé en ce que** le stator (5) présente une fermeture magnétique (7), de manière préférée un paquet de tôles stratifié agencé autour de l'enroulement (6) cylindrique creux sans fer.

9. Moteur électrique à courant continu sans balais implantable (1) selon l'une quelconque des revendications 1 à 8,
**caractérisé en ce que** l'entrefer (17) entre le rotor (2) et le stator (5) permet une circulation de fluide à travers l'entrefer (17), en particulier permet la circulation de sang humain, dans lequel l'entrefer (17) périphérique est de manière préférée d'une taille supérieure à 15 %, en particulier supérieure à 25 %, du rayon du rotor (2).
